# EUROPEAN PATENT APPLICATION

(11) **EP 2 989 959 A1**
(43) Date of publication of application: **02.03.2016**
(21) Application number: 14807165.7
(22) Date of filing: 02.04.2014
(51) Int. Cl.: A61B 1/00, G02B 23/26

(54) **SCANNING ENDOSCOPE**

(30) Priority: 03.06.2013 JP 2013116972
(71) Applicant: OLYMPUS CORPORATION, Shibuya-ku Tokyo 151-0072 (JP)
(72) Inventor: SAKAI, Yuji, Tokyo 151-0072 (JP); WATANABE, Katsushi, Tokyo 151-0072 (JP)
(74) Representative: Gunzelmann, Rainer
(86) International application number: PCT/JP2014/059749
(87) International publication number: WO 2014/196258

(57) **Abstract**

A scanning endoscope includes: a light-guiding section that guides illuminating light for illuminating a subject and emits the illuminating light from a light emission surface; an actuator section provided at an end portion on a light emission side of the light-guiding section, the actuator section vibrating according to supply of a drive signal for scanning the subject; a first covering member covering the end portion and the actuator section; a holding member that is formed so as to have a predetermined length in a direction parallel to a longitudinal direction of the light-guiding section, and can hold a part of the end portion, the part including the light emission surface, and the actuator section in a cantilevered manner in an inner space of the first covering member; a second covering member covering the first covering member; and a fixing portion that fixes the first covering member to the second covering member at a fixing position that is set within a range of the predetermined length and is included in a plane perpendicular to the longitudinal direction of the light-guiding section.

## Description

### Technical Field

The present invention relates to a scanning endoscope, and specifically relates to a scanning endoscope that scans a subject to acquire an image.

### Background Art

For endoscopes in a medical field, various techniques for reduction in diameter of an insertion portion to be inserted into a body cavity of an examinee have been proposed in order to reduce a burden on the examinee. As endoscopes employing such techniques, for example, scanning endoscopes having no solid image pickup device in a part corresponding to the aforementioned insertion portion are conventionally known.

More specifically, a scanning endoscope such as mentioned above is configured to, for example, vibrate an actuator attached to an end portion on the light emission side of an illumination optical fiber that guides illuminating light emitted from a light source section to swing the illumination optical fiber in a predetermined scanning pattern, and thereby perform scanning of a subject within a scanning range corresponding the predetermined scanning pattern. Japanese Patent Application Laid-Open Publication No. 2011-504783 discloses a scanning fiber illuminator having a configuration that is similar to that of the scanning endoscope mentioned above.

A scanning endoscope having a configuration such as mentioned above has a problem that, for example, swinging of a part of an illumination optical fiber on the distal end side relative to a position of an attached actuator (part directly related to scanning of a subject) become unstable as a result of a part of the illumination optical fiber on the proximal end side relative to the position of the attached actuator (part not directly related to scanning of a subject) being swung along with, e.g., vibration of the actuator or disturbance.

However, Japanese Patent Application Laid-Open Publication No. 2011-504783 specifically indicates no approach or the like that can solve the aforementioned problem. Thus, the configuration of the scanning fiber illuminator disclosed in Japanese Patent Application Laid-Open Publication No. 2011-504783 still faces an issue relating to the aforementioned problem of the inability to perform stable scanning of a subject in an originally-intended scanning pattern.

The present invention has been made in view of the aforementioned circumstances, and an object of the present invention is to provide a scanning endoscope that can perform stable scanning of a subject compared to conventional techniques.

### Disclosure of Invention

### Means for Solving the Problem

A scanning endoscope according to an aspect of the present invention includes: a light-guiding section configured to guide illuminating light for illuminating a subject and emit the illuminating light from a light emission surface; an actuator section provided at an end portion on a light emission side of the light-guiding section, the actuator section being configured to vibrate according to supply of a drive signal for scanning the subject in a predetermined scanning pattern; a first covering member having a hollow tubular shape, the first covering member being formed so as to cover a periphery of the end portion and the actuator section; a holding member that is formed so as to have a predetermined length in a direction parallel to a longitudinal direction of the light-guiding section, and is configured to be able to hold a part of the end portion, the part including the light emission surface, and the actuator section in a cantilevered manner in an inner space of the first covering member; a second covering member having a hollow tubular shape, the second covering member being formed so as to cover a periphery of the first covering member; and a fixing portion configured to fix the first covering member to the second covering member at a fixing position that is set within a range of the predetermined length and is included in a plane perpendicular to the longitudinal direction of the light-guiding section.

### Brief Description of the Drawings

Fig. 1 is a diagram illustrating a configuration of a major part of a scanning endoscope system including a scanning endoscope according to an embodiment of the present invention;
Fig. 2 is a diagram illustrating an example of a configuration of a distal end portion of the scanning endoscope according to the embodiment;
Fig. 3 is a cross-sectional view along line III-III in Fig. 2;
Fig. 4 is a diagram illustrating an example of the configuration of the distal end portion of the scanning endoscope according to the embodiment, which is different from the example in Fig. 2;
Fig. 5 is a diagram illustrating an example of the configuration of the distal end portion of the scanning endoscope according to the embodiment, which is different from the examples in Figs. 2 and 4; and
Fig. 6 is a diagram illustrating an example of the configuration of the distal end portion of the scanning endoscope according to the embodiment, which is different from the examples in Figs. 2, 4 and 5.

### Best Mode for Carrying Out the Invention

An embodiment of the present invention will be described below with reference to the drawings.

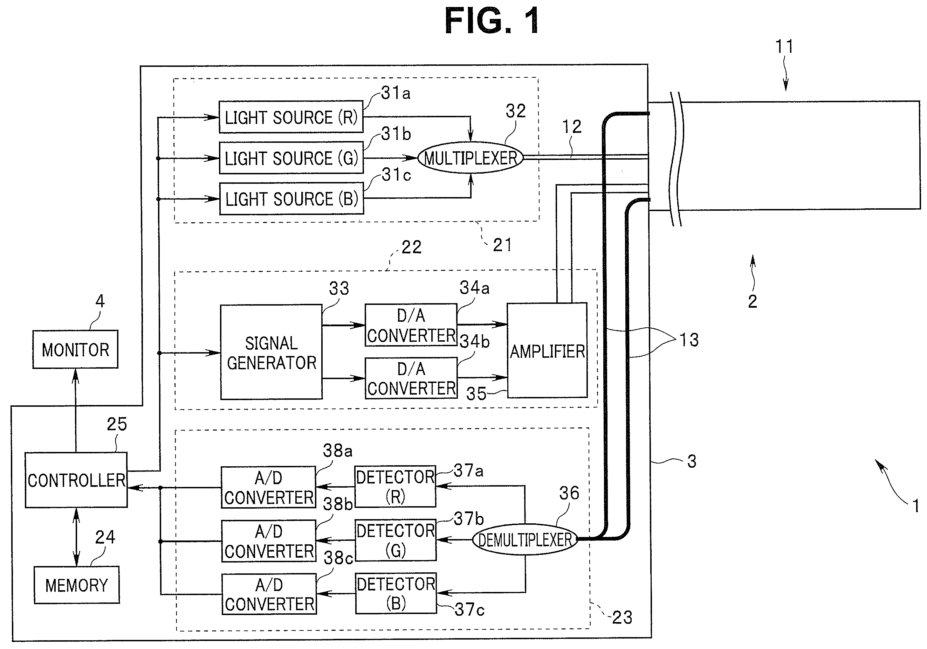

Figs. 1 to 6 relate to an embodiment of the present invention. Fig. 1 is a diagram illustrating a configuration of a major part of a scanning endoscope system including a scanning endoscope according to an embodiment of the present invention.

The scanning endoscope system 1 includes, for example, as illustrated in Fig. 1, a scanning endoscope 2 that can be inserted into a body cavity of an examinee, a main body apparatus 3 connected to the scanning endoscope 2, and a monitor 4 connected to the main body apparatus 3.

The scanning endoscope 2 includes an insertion portion 11 having an elongated cylindrical shape and flexibility. At a proximal end portion of the insertion portion 11, e.g., a non-illustrated connector for detachably connecting the scanning endoscope 2 to the main body apparatus 3 is provided.

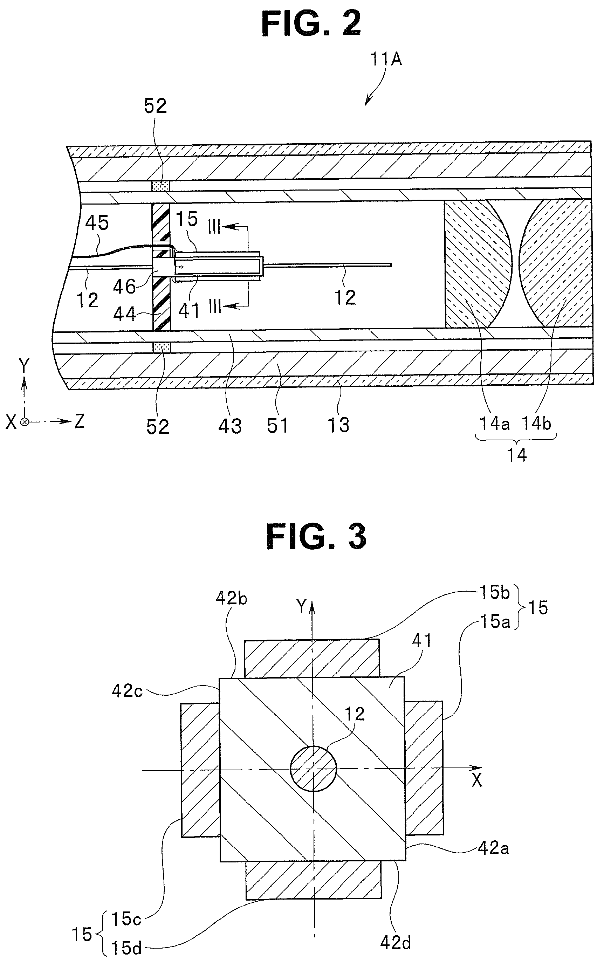

Fig. 2 is a diagram illustrating an example of a configuration of a distal end portion of the scanning endoscope according to the embodiment. As illustrated in Fig. 2, in a distal end portion 11A of the insertion portion 11, an end portion on the light emission side of an illumination optical fiber 12 configured as a light-guiding section that guides illuminating light supplied from the body apparatus 3 and emits the illuminating light from a light emission surface, an end portion on the light entrance side of an light reception optical fiber 13 that receives return light from a subject and guides the return light to the main body apparatus 3, a light collection optical system 14 configured to collect illuminating light from the illumination optical fiber 12 and emit the illuminating light, and an actuator section 15 provided on the end portion on the light emission side of the illumination optical fiber 12, the actuator section 15 being capable of swinging the illumination optical fiber 12 by vibrating according to a drive signal supplied via a plurality of signal wires 45 connected to the main body apparatus 3, are provided. A lens 14a and a lens 14b of the light collection optical system 14 are each formed so as to have positive refractive power.

Also, as illustrated in Fig. 2, in the distal end portion 11A of the insertion portion 11, a ferrule 41, through which the end portion on the light emission side of the illumination optical fiber 12 is disposed so as to extend, includes an outer surface with the actuator section 15 arranged thereon, and a covering member 43 having a hollow tubular shape, a holding member 44 that holds the actuator section 15 and the ferrule 41 in the covering member 43, and the plurality of signal wires 45 are provided.

As illustrated in Fig. 2, the covering member 43 is formed so as to have an inner diameter that enables the covering member 43 to hold the light collection optical system 14 while covering a periphery of the end portion on the light emission side of the illumination optical fiber 12, the actuator section 15 and the ferrule 41.

As illustrated in Fig. 2, the holding member 44 is formed so as to have, for example, a rough ring shape along a plane (hereinafter also referred to as "X-Y plane") perpendicular to a longitudinal direction of the illumination optical fiber 12 (hereinafter also referred to as "Z-axis direction"). Also, the holding member 44 is formed so as to have a length of TH in a direction parallel to the Z-axis direction (thickness), and is attached to an inner peripheral face of the covering member 43 with a side face having the length of TH of the holding member 44 abutting against the inner peripheral face. In other words, in the above-described configuration, the holding member 44 has a roughly tubular shape having a length of TH in the direction parallel to the Z-axis direction.

The holding member 44 includes a hole portion that allows a ferrule proximal end portion 46 to be fitted in, the ferrule proximal end portion 46 being positioned apart on the proximal end side from a position where the actuator section 15 is disposed. In the holding member 44, an insertion hole through which the plurality of signal wires 45 can be inserted is provided. In other words, in the above-described configuration, as a result of the ferrule proximal end portion 46 of the ferrule 41 being fitted in the hole portion of the holding member 44, a part of the end portion on the light emission side of the illumination optical fiber 12, the part including the light emission surface, the actuator section 15 connected to the plurality of signal wires 45, and the ferrule 41 are held in a cantilevered manner in an inner space of the covering member 43.

Also, as illustrated in Fig. 2, in the distal end portion 11A of the insertion portion 11, a covering member 51 having a hollow tubular shape, the covering member 51 being formed so as to have a roughly uniform inner diameter that enables the covering member 51 to cover a periphery of the covering member 43, and a fixing portion 52 for fixing the covering member 43 to the covering member 51 are provided.

As illustrated in Fig. 2, the fixing portion 52 is configured to fix the covering member 43 to the covering member 51 at a fixing position included in the X-Y plane, the fixing position being set within a range of the length TH of the holding member 44. In other words, the fixing portion 52 is configured to fix the covering member 43 to the covering member 51 in an area including a plane that is perpendicular to the longitudinal direction of the illumination optical fiber 12 and extends through the holding member 44.

More specifically, the fixing portion 52 is configured, for example, as a joint member that can joint and fix an outer peripheral face of the covering member 43 and an inner peripheral face of the covering member 51 to each other at a fixing position satisfying the aforementioned conditions. Or, the fixing portion 52 is configured, for example, as an adhesive member such as an adhesive applied to the outer peripheral face of the covering member 43 (or the inner peripheral face of the covering member 51) at the fixing position satisfying the aforementioned conditions. Or, the fixing portion 52 is configured, for example, so as to include a groove portion formed at the fixing position satisfying the aforementioned conditions in the outer peripheral face of the covering member 43, and a protrusion portion formed so as to have a distal end shape that is fitted in, is threadably connected to or is engaged with the groove portion. Here, as long as the fixing portion 52 fixes the covering member 43 to the covering member 51 at the fixing position satisfying the aforementioned conditions, the fixing portion 52 may be provided so as to surround the periphery of the covering member 43 or may be provided on only a part of the periphery of the covering member 43.

Fig. 3 is a cross-sectional view along line III-III in Fig. 2. As illustrated in Fig. 3, the ferrule 41, which serves a joint member, is disposed between the illumination optical fiber 12 and the actuator section 15. More specifically, the ferrule 41 includes, for example, zirconia (ceramic) or nickel.

As illustrated in Fig. 3, the ferrule 41 is formed in a quadrangular prism shape, and includes side faces 42a and 42c perpendicular to an X-axis direction, and side faces 42b and 42d perpendicular to a Y-axis direction. Also, at a center of the ferrule 41, the illumination optical fiber 12 is disposed in a fixed manner.

Note that the ferrule 41 may be formed in a shape other than a quadrangular prism shape as long as the shape is a prism shape.

As illustrated in Fig. 3, the actuator section 15 includes an actuator 15a disposed along the side face 42a, an actuator 15b disposed along the side face 42b, an actuator 15c disposed along the side face 42c, and an actuator 15d disposed along the side face 42d.

Each of the actuators 15a and 15c includes, for example, a piezoelectric element, and is configured to vibrate according to a drive signal outputted from an amplifier 35 via a D/A converter 34a of a driver unit 22.

Each of the actuators 15b and 15d includes, for example, a piezoelectric element, and is configured to vibrate according to a drive signal outputted from the amplifier 35 via a D/A converter 34b of the driver unit 22.

The main body apparatus 3 includes a light source unit 21, the driver unit 22, a detection unit 23, a memory 24 and a controller 25.

The light source unit 21 has a function as a light source section that supplies illuminating light for illuminating a subject. More specifically, as illustrated in Fig. 1, the light source unit 21 includes a light source 31a, a light source 31b, a light source 31c and a multiplexer 32.

The light source 31a includes, for example, a laser light source, and is configured to, upon the light source 31a being turned on by control performed by the controller 25, emit light in a red wavelength band (hereinafter also referred to as "R light") to the multiplexer 32.

The light source 31b includes, for example, a laser light source, and is configured to, upon the light source 31b being turned on by control performed by the controller 25, emit light in a green wavelength band (hereinafter also referred to as "G light") to the multiplexer 32.

The light source 31 c includes, for example, a laser light source, and is configured to, upon the light source 31 c being turned on by control performed by the controller 25, emit light in a blue wavelength band (hereinafter also referred to as "B light") to the multiplexer 32.

The multiplexer 32 is configured to be able to multiplex the R light emitted from the light source 31 a, the G light emitted from the light source 31 b, and the B light emitted from the light source 31c and supply the multiplexed lights to a light entrance surface of the illumination optical fiber 12.

As illustrated in Fig. 1, the driver unit 22 includes a signal generator 33, the D/A converters 34a and 34b and the amplifier 35.

The signal generator 33 is configured to generate a drive signal for swinging the illumination optical fiber 12 based on control performed by the controller 25 and output the drive signal to the D/A converters 34a and 34b.

Each of the D/A converter 34a and 34b is configured to convert the digital drive signal outputted from the signal generator 33 into an analog drive signal and output the analog drive signal to the amplifier 35.

The amplifier 35 is configured to amplify the drive signals outputted from the D/A converter 34a and 34b and output the drive signals the actuator section 15.

As illustrated in Fig. 1, the detection unit 23 includes a demultiplexer 36, detectors 37a, 37b and 37c and A/D converters 38a, 38b and 38c.

The demultiplexer 36 includes, e.g., a dichroic mirror, and is configured to split return light emitted from a light emission surface of the light reception optical fiber 13 into light of respective color components of R (red), G (green) and B (blue) and emit the light of the respective color components to the respective detectors 37a, 37b and 37c.

The detector 37a is configured to detect an intensity of the R light emitted from the demultiplexer 36, generate an analog R signal according to the detected intensity of the R light and output the analog R signal to the A/D converter 38a.

The detector 37b is configured to detect an intensity of the G light emitted from the demultiplexer 36, generate an analog G signal according to the detected intensity of the G light and output the analog G signal to the A/D converter 38b.

The detector 37c is configured to detect an intensity of the B light emitted from the demultiplexer 36, generate an analog B signal according to the detected intensity of the B light and output the analog B signal to the A/D converter 38c.

The A/D converter 38a is configured to convert the analog R signal outputted from the detector 37a into a digital R signal and output the digital R signal to the controller 25.

The A/D converter 38b is configured to convert the analog G signal outputted from the detector 37b into a digital G signal and output the digital G signal to the controller 25.

The A/D converter 38c is configured to convert the analog B signal outputted from the detector 37c into a digital B signal and output the digital B signal to the controller 25.

In the memory 24, e.g., a control program for control of the main body apparatus 3 is stored in advance.

The controller 25 includes, e.g., a CPU, and is configured to read the control program stored in the memory 24 and perform control of the light source unit 21 and the driver unit 22 based on the read control program. In other words, the actuator section 15 can swing the illumination optical fiber 12 so that a position of the illuminating light applied to a subject draws a trajectory according to a predetermined scanning pattern (for example, a spiral shape or a Lissajous figure) by vibrating based on a drive signal supplied from the driver unit 22 according to the aforementioned control performed by the controller 25.

The controller 25 is configured to generate an image based on the R signal, the G signal and the B signal outputted from the detection unit 23 and display the generated image on the monitor 4.

Next, operation of the scanning endoscope system 1 including the scanning endoscope 2 according to the present embodiment will be described.

After power is supplied to the respective sections of the scanning endoscope system 1, based on the control program stored in the memory 24, the controller 25 controls the light source unit 21 to switch the light sources 31a and 31b and the light source 31c on from an off state, and controls the driver unit 22 to cause the signal generator 33 to output a drive signal for swinging the illumination optical fiber 12 in a predetermined scanning pattern. As a result of the control performed by the controller 25, a drive signal is supplied from the driver unit 22 to the actuator section 15, the actuator section 15 vibrates according to the supplied drive signal, the illumination optical fiber 12 is swung in the predetermined scanning pattern, and mixture light of R light, G light and B light is emitted as illuminating light from the light emission surface of the illumination optical fiber 12.

Here, in the above-described configuration of the distal end portion 11 A, the fixing portion 52 for fixing the covering member 43 to the covering member 51 is provided in alignment with a position where the holding member 44 for holding the actuator section 15 is provided. Thus, the above-described configuration of the distal end portion 11A enables prevention of a part of the illumination optical fiber 12 on the proximal end side relative to the position where the covering member 43 is fixed by the fixing portion 52 (part that neither includes the light emission surface nor directly related to scanning of a subject) from being swung along with vibration of the actuator section 15. Consequently, the scanning endoscope 2 including the distal end portion 11A enables only the part of the illumination optical fiber 12 on the distal end side relative to the position where the covering member 43 is fixed by the fixing portion 52 (part that includes the light emission surface and is directly related to scanning of a subject) to be swung according to vibration of the actuator section 15, that is, enables stable scanning of a subject.

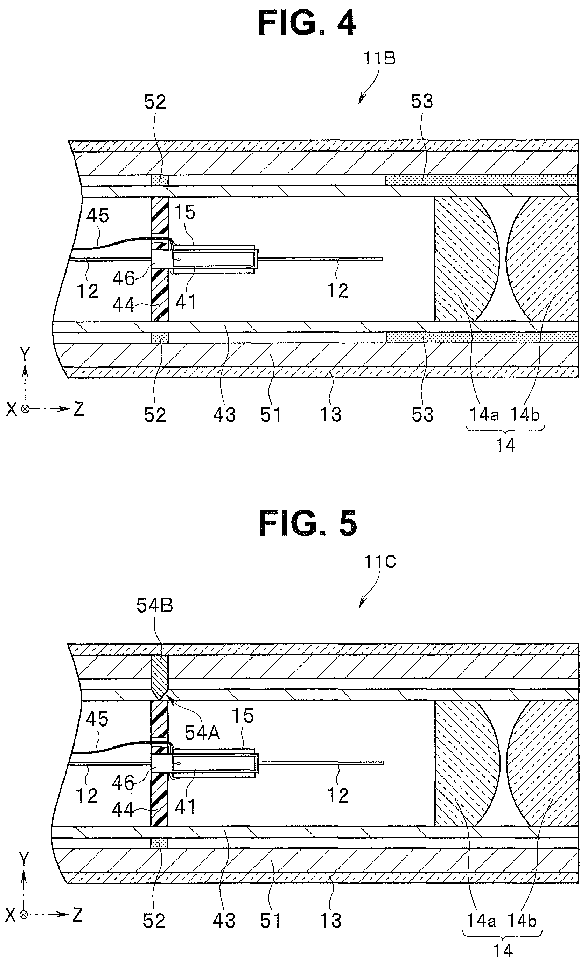

In the present embodiment, a scanning endoscope 2 may include, for example, a distal end portion 11B, which is illustrated in Fig. 4, instead of the distal end portion 11A illustrated in Fig. 2. Fig. 4 is a diagram illustrating an example of the configuration of the distal end portion of the scanning endoscope according to the embodiment, which is different from the example in Fig. 2. Note that in the below description, for simplicity, detailed description of parts having configurations similar to those of the distal end portion 11 A is omitted, and the description will be provided mainly on parts that are different from those of the distal end portion 11A.

As illustrated in Fig. 4, the distal end portion 11B is configured by further providing a distal end fixing portion 53 to the distal end portion 11A.

The distal end fixing portion 53 is provided on the outer peripheral face of the covering member 43, and is formed so as to fix the covering member 43 to the covering member 51 at a fixing position where the distal end fixing portion 53 surrounds at least a periphery of positions where the lens 14a and the lens 14b of the light collection optical system 14 are disposed.

Thus, the scanning endoscope 2 including the distal end portion 11B enables stable scanning of a subject with the covering member 43 firmly fixed to the covering member 51.

Also, in the present embodiment, a scanning endoscope 2 may include, for example, a distal end portion 11C, which is illustrated in Fig. 5, instead of the distal end portion 11A illustrated in Fig. 2. Fig. 5 is a diagram illustrating an example of the configuration of the distal end portion of the scanning endoscope according to the embodiment, which is different from the examples in Figs. 2 and 4.

As illustrated in Fig. 5, the distal end portion 11C is configured by replacing a part of the fixing portion 52 in the distal end portion 11A with a combination of a groove portion 54A provided in the outer peripheral face of the covering member 43 and a protrusion portion 54B that is disposed so as to project from the inner peripheral face of the covering member 51 and has a distal end shape that is fitted in, threadably connected to or engaged with the groove portion 54A.

Thus, the scanning endoscope 2 including the distal end portion 11C enables stable scanning of a subject with the covering member 43 firmly fixed to the covering member 51.

Also, the scanning endoscope 2 including the distal end portion 11C enables easy recognition of the covering member 43 being fixed to the covering member 51 at a position where the groove portion 54A and the protrusion portion 54B are provided. As a result, the scanning endoscope 2 including the distal end portion 11C enables reduction in burden of work relating to alignment for fixing the covering member 43 to the covering member 51.

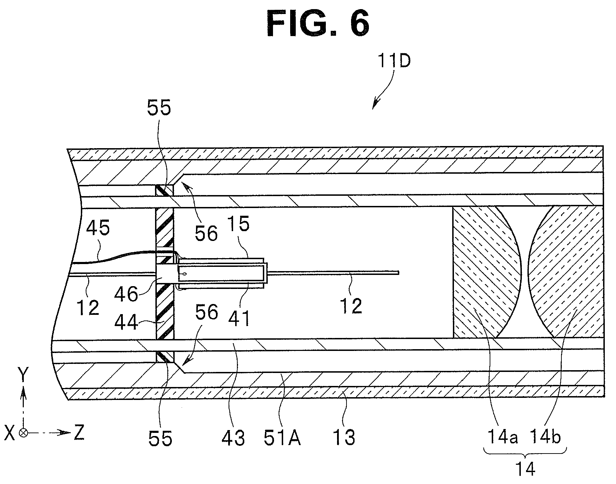

Also, in the present embodiment, the scanning endoscope 2 may include, for example, a distal end portion 11D, which is illustrated in Fig. 6, instead of the distal end portion 11A illustrated in Fig. 2. Fig. 6 is a diagram illustrating an example of the configuration of the distal end portion of the scanning endoscope according to the embodiment, which is different from the examples in Figs. 2, 4 and 5.

As illustrated in Fig. 6, the distal end portion 11D is configured by replacing the covering member 51 in the distal end portion 11A with a covering member 51 A and replacing the fixing portion 52 in the distal end portion 11A with an adhesive 55.

The covering member 51A is formed in a hollow tubular shape that can cover the periphery of the covering member 43, with a tapered portion 56 provided inside. Also, the covering member 51A is formed in such a manner that an inner diameter on the distal end side relative to the tapered portion 56 is larger than an inner diameter on the proximal end side relative to the tapered portion 56.

The adhesive 55 is applied to the fixing position that is set within the range of the length TH of the holding member 44 and is included in the XY plane so as to bond and fix the covering member 43 to the covering member 51. More specifically, the adhesive 55 is applied to, for example, as illustrated in Fig. 6, an area of the outer peripheral face of the covering member 43 or the inner peripheral face of the covering member 51A, the area being positioned on the proximal end side relative to the tapered portion 56 and being adjacent to the tapered portion 56.

The tapered portion 56 is provided adjacent to the distal end side of the area to which the adhesive 55 is applied. In other words, the tapered portion 56 is provided adjacent to the distal end side relative to the fixing position for bonding and fixing the covering member 43 to the covering member 51 A.

Thus, the scanning endoscope 2 including the distal end portion 11D enables stable scanning of a subject.

Also, the scanning endoscope 2 including the distal end portion 11D enables a position where the adhesive 55 is to be applied to be easily recognized based on the position where the tapered portion 56 is provided. Consequently, the scanning endoscope 2 including the distal end portion 11D enables reduction in burden of work relating to alignment for bonding and fixing the covering member 43 to the covering member 51 A.

The present invention is not limited to each of the embodiments described above, and it should be understood that various modifications and applications are possible without departing from the spirit of the present invention.

The present application is filed claiming priority from Japanese Patent Application No. 2013-116972 filed in Japan on June 3, 2013, the disclosure of which is incorporated in the description, the claims and the drawings of the present application by reference.

## Claims

1. A scanning endoscope comprising:
a light-guiding section configured to guide illuminating light for illuminating a subject and emit the illuminating light from a light emission surface;
an actuator section provided at an end portion on a light emission side of the light-guiding section, the actuator section being configured to vibrate according to supply of a drive signal for scanning the subject in a predetermined scanning pattern;
a first covering member having a hollow tubular shape, the first covering member being formed so as to cover a periphery of the end portion and the actuator section;
a holding member that is formed so as to have a predetermined length in a direction parallel to a longitudinal direction of the light-guiding section, and is configured to be able to hold a part of the end portion, the part including the light emission surface, and the actuator section in a cantilevered manner in an inner space of the first covering member;
a second covering member having a hollow tubular shape, the second covering member being formed so as to cover a periphery of the first covering member; and
a fixing portion configured to fix the first covering member to the second covering member at a fixing position that is set within a range of the predetermined length and is included in a plane perpendicular to the longitudinal direction of the light-guiding section.

2. The scanning endoscope according to claim 1, further comprising a joint member, through which the end portion is disposed so as to extend, the joint member including an outer surface with the actuator section arranged on the outer surface,
wherein the holding member includes a hole portion that allows a proximal end portion of the joint member positioned apart from a position where the actuator section is arranged, to be fitted in the hole portion.

3. The scanning endoscope according to claim 1, further comprising:
a light collection optical system provided in the end portion, the light collection optical system being configured to collect the illuminating light from the light emission surface and emit the illuminating light to the subject; and
a distal end fixing portion that is provided on an outer peripheral face of the first covering member and is formed so as to fix the first covering member to the second covering member at a fixing position where the distal end fixing portion surrounds at least a periphery of a position where the light collection optical system is arranged.

4. The scanning endoscope according to claim 1, wherein a part of the fixing portion includes a combination of a groove portion provided in an outer peripheral face of the first covering member and a protrusion portion disposed so as to project from an inner peripheral face of the second covering member, the protrusion portion having a distal end shape that is fitted in, is threadably connected to or is engaged with the groove portion.

5. The scanning endoscope according to claim 1, wherein
the second covering member is formed in such a manner that an inner diameter on a distal end side relative to a tapered portion provided according to the fixing position is larger than an inner diameter on a proximal end side relative to the tapered portion; and
the first covering member is bonded and fixed to the second covering member by the fixing portion configured as an adhesive applied to an area that is positioned on the proximal end side relative to the tapered portion and is adjacent to the tapered portion.
